# EUROPEAN PATENT SPECIFICATION

(11) **EP 2 172 223 B1**
(45) Date of publication and mention of the grant of the patent: **16.12.2020**
(21) Application number: 08765782.1
(22) Date of filing: 20.06.2008
(51) Int. Cl.: A61K 9/107, A61K 9/14, A61K 45/06, A61K 31/122, A61K 31/07, A61K 31/355, A61K 31/59, A61K 36/8962, A61K 36/9068, A61K 36/752, A61K 36/54, A61K 36/71, A61K 36/53, A61K 36/236, A23L 33/15, A23L 33/105, A61P 3/02

(54) **COMPOSITION CONTAINING PHYSIOLOGICALLY ACTIVE SUBSTANCE**
EINE PHYSIOLOGISCH WIRKSAME SUBSTANZ ENTHALTENDE ZUSAMMENSETZUNG
COMPOSITION CONTENANT UNE SUBSTANCE PHYSIOLOGIQUEMENT ACTIVE

(30) Priority: 22.06.2007 JP 2007165175; 05.11.2007 US 996158 P
(43) Date of publication of application: 07.04.2010
(73) Proprietor: Kaneka Corporation, Osaka (JP)
(72) Inventor: IKEHARA, Toshinori, Takasago-shi Hyogo 676-8688 (JP); SAKOGAWA, Takayuki, Takasago-shi Hyogo 676-8688 (JP)
(74) Representative: J A Kemp LLP
(86) International application number: PCT/JP2008/061362
(87) International publication number: WO 2009/001786

(56) References cited:
- EP-A1- 1 782 803
- EP-A1- 2 098 222
- JP-A- H0 597 672
- JP-A- H0 717 860
- JP-A- S5 951 214
- JP-A- H02 204 417
- JP-A- H04 112 824
- JP-A- H07 506 366
- JP-A- S55 147 219
- JP-A- S58 109 412
- JP-A- 2000 119 686
- JP-A- 2000 212 066
- JP-A- 2003 176 233
- JP-A- 2003 183 692
- JP-A- 2003 238 396
- US-A- 4 751 241
- DATABASE WPI Week 200359 Thomson Scientific, London, GB; AN 2003-621876 XP002681909, & JP 2003 183692 A (KAO CORP) 3 July 2003 (2003-07-03)
- DATABASE CAPLUS, [Online] 4 June 2007 (2007-06-04), SHAO BIN ET AL: "Dry powder composition containing coenzyme Q10 and its preparation", XP008118662, retrieved from CAPLUS Database accession no. 2007-597834 147-102030 -& CN 1 969 833 A (ZHEJIANG MEDICINE CO LTD XINCH [CN]) 30 May 2007 (2007-05-30)

## Description

### [Technical Field]

The present invention relates to an emulsified powder as defined herein, comprising coenzyme Q10 as a bioactive substance. More specifically, the present invention relates to an emulsified powder comprising said bioactive substance, having powder characteristics allowing easy handling and high oral absorbability.

### [Background Art]

For oil-soluble bioactive substances, their processing into tablets and their addition to foods such as beverages are difficult, and their absorbability in oral ingestion is low in quite a few cases. For example, coenzyme Q10 is an oil-soluble crystalline powder having a melting point of about 48°C, and is known to have a very low absorbability in oral ingestion because it is hardly soluble in water.

Coenzyme Q10 is a physiological component present as a constituent component of the mitochondrial electron transport system in the cell of the living body. It functions as a transport component in the electron transport system by repeating oxidation and reduction in the living body. Coenzyme Q10 is known to show energy production, membrane stabilization and antioxidant activity in the living body, and has a high degree of usability. Coenzyme Q10 occurs in two forms, the oxidized form and the reduced form, and it is known that, in the living body, usually about 40 to 90% of the coenzyme exists in the reduced form. Of coenzymes Q10, oxidized coenzyme Q10 (aka. ubiquinone or ubidecarenone) is widely used in the pharmaceutical field as a drug for congestive heart failure. Besides the pharmaceutical use, it is widely used as an agent for oral preparation and a skin preparation, as a nutritional product or a nutritional supplement, like vitamin. More recently, oxidized coenzyme Q10 has been approved for use as a food, and it is drawing attention as a material for health foods. It is known, however, that oxidized coenzyme Q10 poses problems of poor powder fluidity and tableting trouble when used as is for hard capsules and tableting. To solve these problems, a wide variety of technical proposals have been made to date. For example, it has been proposed to prepare an emulsified product containing coenzyme Q10 by using a synthetic emulsifier such as glycerol fatty acid ester, sucrose fatty acid ester and the like, and powderize the product by a spray dry method and the like (patent references 1 - 3). Such production method achieves superior emulsion stability. However, it requires addition of a large amount of excipient for production of a dry powder and, as a result, the content of coenzyme Q10 in the obtained powder cannot be high.

Although solubilized powders of coenzyme Q10 and methods of preparing the same have been developed to date, many of them have only limited uses, such as for either a preparation such as tablets or a part of foods. In this situation, there has been a demand for a powder preparation of high contents of coenzyme Q10 that can be utilized not only for tablets and hard capsules, but also for all kinds of foods, including beverages, confectionery, and milk products, and that have excellent water-dispersibility, water-solubility and workability.

For the same reason, a powder preparation superior in water-dispersibility/solubility and workability, which contains a further oil-soluble or hardly water-soluble bioactive substance (e.g., oil-soluble vitamins, hydrophobic polyphenols and the like) other than coenzyme Q10 at a high level, is desired.

As a prior art for powderizing such an oil component, a technique for powderizing edible fats and oils can be mentioned. For example, although a technique for containing an oil component, gum arabic and saccharide at a particular ratio is disclosed, since the ratio of saccharide is high, application to a supplement material is often hesitated in view of problematic sweetness and high calorie (patent reference 4). In addition, while a powder of fat and oil containing unsaturated fatty acid, which uses sugar alcohol, is disclosed, this technique aims to improve oxidization stability of unsaturated fatty acid (patent reference 5).

Patent Reference 6 discloses a particulate composition wherein an oil component (A) comprising a poorly water-soluble bioactive substance is polydispersed while forming a domain in a matrix comprising a water-soluble excipient comprising a water-soluble polymer as a main component, and wherein the sphericity is not less than 0.9.

Patent Reference 7 discloses a coenzyme Q₁₀-containing liquid composition comprising coenzyme Q₁₀, glycerin, a water-soluble substance consisting of octenylsuccinate starch, dextrin and glycerin, which is obtained by dispersing and emulsifying coenzyme Q₁₀ in aqueous liquid containing the water-soluble substance. The liquid composition may be dried to prepare a coenzyme Q₁₀-containing solid composition.

Patent Reference 8 disloses a dry powder composition containing coenzyme Q₁₀, excipient (sucrose, glucose, maltodextrin or microcrystalline cellulose), embedded material (modified starch, arabic gum, casein, caseinate, gelatin, PVP or hydroxymethyl cellulose), anti-oxidant, emulsifying agent and water.

Patent Reference 9 discloses an oral composition which comprises a polyglycerol ester of an unsaturated fatty acid and a drug, such as cyclandelate, which is very slightly soluble in water.
[patent reference 1] JP-A-59-51214
[patent reference 2] JP-A-2000-212066
[patent reference 3] JP-A-2003-238396
[patent reference 4] JP-A-2000-119686
[patent reference 5] JP-A-2003-183692
[patent reference 6] EP-A-2098222
[patent reference 7] EP-A-1782803
[patent reference 8] CN-A-1969833
[patent reference 9] US-A-4751241

### [Disclosure of the Invention]

### [Problems to be Solved by the Invention]

To solve the above-mentioned problems, the present invention proposes an emulsified powder containing coenzyme Q10 as a bioactive substance applicable to the fields of general foods, food with nutrient function claims, food for specified health uses, nutritional supplement, nutritional product, animal drug, drinks, feed, cosmetic, quasi-drug, pharmaceutical product, therapeutic drug, and prophylactic drug and superior not only in the water-dispersibility/solubility but also in the recovery rate during production, workability such as powder flowability, as well as tabletability.

### [Means of Solving the Problems]

The present inventors have conducted intensive studies in an attempt to solve the above-mentioned problems and found that an emulsified powder comprising an oil component containing coenzyme Q10 as a bioactive substance, which is dispersed in a matrix comprising a water-soluble polymer, a saccharide as defined herein other than polysaccharides and a surfactant at a particular ratio, is a composition superior in the water-dispersibility/solubility, workability and tabletability, which resulted in the completion of the present invention.

Accordingly, the present invention provides the following.
[1] A bioactive substance-containing emulsified powder comprising 1 - 70 wt% of an oil component (A) comprising coenzyme Q10 as a bioactive substance, 20 - 97.9 wt% of a water-soluble polymer (B), 1 - 20 wt% of a saccharide (C) other than polysaccharides, wherein the saccharide (c) is at least one kind of sugar alcohol selected from the group consisting of sorbitol, mannitol, xylitol, erythritol, maltitol, lactitol and palatinit, and 0.1 - 30 wt% of a surfactant (D).
[2] The powder of the aforementioned [1], wherein the oil component (A) further comprises at least one further bioactive substance selected from the group consisting of oil-soluble vitamins, plant extract and carotenoids.
[3] The powder of the aforementioned [1], wherein the water-soluble polymer (B) is at least one kind selected from the group consisting of gum arabic, gum ghatti, gelatine, agar, starch, modified starch, pectin, carrageenan, casein, dried albumen, curdlan, alginic acids, soybean polysaccharide, pullulan, celluloses, xanthan gum, carmellose salt and polyvinylpyrrolidone.
[4] The powder of the aforementioned [1], wherein the surfactant (D) is at least one kind selected from the group consisting of glycerol fatty acid esters, sucrose fatty acid esters, sorbitan fatty acid esters, lecithins and saponins.
[5] The powder of the aforementioned [2], wherein the oil-soluble vitamin is at least one kind selected from the group consisting of vitamin A, vitamin D, vitamin E, vitamin K and derivatives thereof.
[6] The powder of the aforementioned [2], wherein the plant extract is at least one kind selected from the group consisting of licorice, turmeric, perilla, clove, cinnamon, ginger, lemongrass, peppermint, dokudami (Houttuyania cordata), coix seed, rice bran, cornflower, fennel, boxthorn, zanthoxylum, nasturtium, dioscorea, sanryo, kinkaran (Tinospora capillipes), *amachazuru* (Gynostema pentaphyllum), thuja, hakutouou (Pulsatilla chinensis), parsley, onion, nutmeg, wild-rice, gluten feed, *konnyaku tobiko* (konnyaku byproduct powder), paprika, horseradish, lemon, capsicum, sesame, spearmint and leaf mustard.
[7] The powder of the aforementioned [2], wherein the carotenoid is at least one kind selected from the group consisting of carotenes, xanthophylls, and derivatives thereof.
[8] The powder of any of the aforementioned [1] to [7],
   wherein the oil component (A) comprises 5 - 100 wt% of a bioactive substance and 0 - 95 wt% of fat and oil.
[9] The powder of the aforementioned [8], wherein the fat and oil is medium chain triglyceride.
[10] A food, pharmaceutical product, cosmetic or feed comprising the powder of any one of the aforementioned [1] to [9] .

### [Effect of the Invention]

According to the present invention, a bioactive substance-containing powder as defined herein, superior in water-dispersibility/solubility, workability and tabletability can be provided, which is advantageous for the production of food, food with nutrient function claims, food for specified health uses, nutritional supplement, nutritional product, animal drug, drinks, feed, cosmetic, quasi-drug, pharmaceutical product, therapeutic drug, and prophylactic drug.

### [Best Mode for Carrying out the Invention]

The bioactive substance-containing powder of the present invention is an emulsified powder containing 1 - 70 wt% of an oil component (A) containing coenzyme Q10 as a bioactive substance, 20 - 97.9 wt% of water-soluble polymer (B), 1 - 20 wt% of a saccharide (C) as defined herein other than polysaccharides and 0.1 - 30 wt% of surfactant (D) (hereinafter sometimes to be referred to as a powder of the present invention). Specifically, it is an emulsified powder comprising an oil component (A) containing coenzyme Q10 as a bioactive substance dispersed in a matrix comprising of water-soluble polymer (B), a saccharide (C) as defined herein other than polysaccharide and surfactant (D). The emulsified powder here means a powder that becomes an oil-in-water emulsion when dissolved in water.

As the coenzyme Q10 to be used in the present invention, both an oxidized coenzyme Q10 represented by the following formula (1) and a reduced coenzyme Q10 represented by the following formula (2) can be used, and a mixture thereof can also be used. 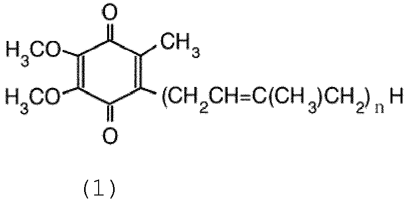 wherein n=10. 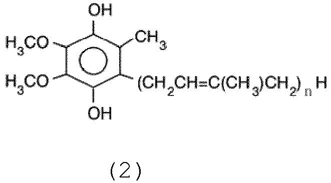 wherein n=10.

The oil-soluble vitamins suitable for use as a further bioactive substance in the present invention include vitamin A, vitamin D, vitamin E such as tocophenol and tocotrienol, vitamin K, and mixtures thereof.

The plant extracts suitable for use as a further bioactive substance in the present invention include hydrophobic plant extracts obtained by extracting a general edible plant with an organic solvent. Examples thereof include a hydrophobic extract obtained by extracting a plant such as licorice, turmeric, perilla, clove, cinnamon, ginger, lemongrass, peppermint, *dokudami* (Houttuyania cordata), coix seed, rice bran, cornflower, fennel, boxthorn, zanthoxylum, nasturtium, dioscorea, sanryo, kinkaran (Tinospora capillipes), *amachazuru* (Gynostema pentaphyllum), thuja, hakutouou (Pulsatilla chinensis), parsley, onion, nutmeg, wild-rice, gluten feed, *konnyaku tobiko* (konnyaku byproduct powder), paprika, horseradish, lemon, capsicum, sesame, spearmint, and leaf mustard or a plant processed product with an organic solvent such as ethanol, acetone, and hexane. The active ingredient of a hydrophobic extract is, for example, a polyphenol or a terpene. Of these, a preferable plant extract is an ethanol extract of licorice, which contains licorice polyphenol as a main ingredient.

Carotenoids suitable for use as a further bioactive substance in the present invention include carotenes, xanthophylls, and derivatives thereof; specifically, carotenes are exemplified by α carotene, β carotene, γ carotene, δ carotene, ε carotene, and licopene, and xanthophylls are preferably exemplified by lutein, zeaxanthin, canthaxanthin, fucoxanthin, anthraxanthin, violaxanthin, and astaxanthin.

The emulsified powder of the present invention contains, as an oil component, the above-mentioned bioactive substance alone or as a mixture with fat and oil. That is, the oil component (A) containing coenzyme Q10 as a bioactive substance and optionally at least one further bioactive substance selected from oil-soluble vitamins, plant extract and carotenoids in the present invention may be any of (1) said bioactive substance(s) alone, and (2) a mixture of said bioactive substance(s) and fat and oil. When the oil component (A) is (2), the oil component is preferably visually uniformly mixed at a temperature at which the bioactive substance is dissolved (for example, when the bioactive substance is oxidized coenzyme Q10, it is melted by heating at 50°C or above).

The fat and oil used when oil component (A) is (2) is not particularly limited and, for example, it may be natural fat and oil from plant or animal, or synthetic fat and oil, processed fat and oil. Preferably, it is acceptable for food, cosmetic or pharmaceutical agent. Examples of the vegetable fat and oil include coconut oil, palm oil, palm kernel oil, flaxseed oil, camellia oil, brown rice germ oil, rape seed oil, rice oil, peanuts oil, corn oil, wheat germ oil, soy bean oil, perilla oil, cottonseed oil, sunflower kerel oil, kapok oil, evening primrose oil, shea butter, sal butter, cacao butter, sesame oil, safflower oil, olive oil, pomegranate oil, and bitter gourd oil, and examples of animal fats and oils include lard, milk fat, fish oil, and beef fat. In addition, medium chain triglyceride (MCT) wherein each fatty acid has a carbon number of 6 - 12, preferably 8 - 12, fats and oils obtained by processing them by fractionation, hydrogenation, transesterification etc. (e.g., hydrogenated oil) and partial glycerides thereof can also be mentioned. Needless to say, a mixture of them may be used. Particularly, MCT is preferable since it can efficiently dissolve a bioactive substance and is superior in easy handling, and odor.

Besides the above-mentioned, oil-soluble components such as solid fat, fatty acid and ester derivative thereof can be contained in oil component (A) according to various objects in the present invention.

Examples of the aforementioned solid fat include wax for food such as bees wax, rhus succedanea fruit wax, candelilla wax, rice bran wax, carnauba wax, and snow wax. Examples of the aforementioned fatty acid and ester derivatives thereof include saturated fatty acids such as caprylic acid, capric acid, lauric acid, myristic acid, palmitic acid, stearic acid, and behenic acid; unsaturated fatty acids such as oleic acid, linoleic acid, conjugated linoleic acid, linolenic acid, punicic acid, docosahexaenoic acid, docosapentaenoic acid, and eicosapentaenoic acid; special fatty acids having an SS bond in the molecular structure thereof, such as α lipoic acid, and esters thereof, for example, methyl esters and ethyl esters thereof.

In the emulsified powder of the present invention, the composition of the oil component (A) containing coenzyme Q10 as a bioactive substance and optionally at least one at least one further bioactive substance selected from oil-soluble vitamins, plant extract and carotenoids is generally 5 - 100 wt% of said bioactive substance and 0 - 95 wt% of fat and oil, preferably 10 - 100 wt% of said bioactive substance and 0 - 90 wt% of fat and oil, more preferably 30 - 100 wt% of said bioactive substance and 0 - 70 wt% of fat and oil. A content of a bioactive substance in oil component (A) of less than 5 wt% is unpreferable since the content of the bioactive substance in the finally-obtained powder decreases, and oral administration of a given amount of the bioactive substance requires ingestion of a large amount of the powder. The mixing ratio of said bioactive substance and fat and oil in oil component (A) is appropriately determined according to the property of the bioactive substance. The oil component containing bioactive substance (A) is preferably coenzyme Q10 alone.

In the present invention, the content of the above-mentioned oil component (A) containing coenzyme Q10 as a bioactive substance and optionally at least one further bioactive substance selected from oil-soluble vitamins, plant extract and carotenoids in a bioactive substance-containing powder is within the range of 1 - 70 wt%, preferably 5 - 60 wt%, more preferably 10 - 50 wt%. When the content of oil component (A) in the powder is less than 1 wt%, oral administration of a given amount of the bioactive substance requires ingestion of a large amount of the powder. On the other hand, a content of oil component (A) in the powder of more than 70 wt% is unpreferable since the recovery rate and powder flowability of the powder are degraded.

Water-soluble polymer (B) to be used for the powder of the present invention should be acceptable for use as food, cosmetic, or pharmaceutical product, with preference given to those particularly acceptable for food. For example, water-soluble polymer containing amino acid and/or sugar as main ingredients such as gum arabic, gum ghatti, guar gum, gelatine, agar, starch, modified starch, pectin, carrageenan, casein (casein and salts thereof), dried albumen, curdlan, alginic acids, soybean polysaccharides, pullulan, celluloses, xanthan gum, carmellose salt (for example carmellose sodium, carmellose calcium), sugar ester of higher fatty acid, tragacanth, milk, polyvinylpyrrolidone can be used singly or in a mixture of two or more kinds thereof. Of these, gum arabic, gum ghatti, gelatine, agar, starch, modified starch, pectin, carrageenan, casein, dried albumen, curdlan, alginic acids, soybean polysaccharides, pullulan, celluloses, xanthan gum, carmellose salt and polyvinylpyrrolidone are preferable. Gum arabic, gum ghatti, gelatine, soybean polysaccharides, modified starch and casein are more preferably used and gum arabic or gum ghatti is most preferably used because a powder simultaneously having excellent water-dispersibility/solubility, workability and tabletability can be achieved.

In the present invention, the content of the above-mentioned water-soluble polymer (B) in the bioactive substance-containing powder is within the range of 20 - 97.9 wt%, preferably 20 - 90 wt%, more preferably 30 - 80 wt%, most preferably 30 - 70 wt%. When the content of the water-soluble polymer is less than 20 wt%, emulsifiability becomes insufficient.

Saccharide (C) other than polysaccharide to be used for the powder of the present invention is sugar alcohol, and polysaccharides are not included, though a small amount of polysaccharides may be contained as other component. Here, polysaccharide means 10 or more monosaccharides bonded to each other, such as dextrin, starch and the like. The saccharides other than polysaccharides consist of at least one kind of sorbitol, mannitol, xylitol, erythritol, maltitol, lactitol and palatinit. Sorbitol, mannitol, xylitol and erythritol are more preferable. Most preferred is erythritol, which is a natural carbohydrate contained in fruit of white grape, mushroom, and fermented foods such as wine, *sake,* soy sauce, *miso* and the like. Erythritol is a noncalonic sugar alcohol industrially produced by a fermentation method using glucose as a starting material, and a small amount thereof shows a most superior flowability-improving effect for an powder.

In the present invention, the content of the above-mentioned saccharide (C) other than polysaccharide in the bioactive substance-containing powder is within the range of 1 - 20 wt%, preferably 5 - 20 wt%. When the content of the saccharide (C) other than polysaccharide is less than 1 wt%, the flowability of the powder is insufficient, and when it exceeds 20 wt%, the flowability of the powder can be improved but the taste is unpreferable.

The surfactant (D) to be used for the powder of the present invention should be acceptable for foods, cosmetics and pharmaceutical products, with particular preference given to those acceptable for foods. Particularly, a hydrophilic surfactant is preferable since an oil component (A) containing coenzyme Q10 as a bioactive substance can be stably processed into an emulsified powder, and a powder superior in water-dispersibility/solubility, workability and tabletability, which is the object of the present invention, can be obtained. For example, a surfactant having an HLB of not less than 6, preferably not less than 8, more preferably not less than 10, can be used. Examples of such surfactant include glycerol fatty acid esters, sucrose fatty acid esters, sorbitan fatty acid esters, lecithins and saponins. They can be used alone or in a mixture of two or more kinds thereof in the present invention.

As the aforementioned glycerol fatty acid esters, for example, fatty acid and organic acid esters of monoglycerol, polyglycerol fatty acid esters, polyglycerin condensed ricinoleate can be mentioned. As the fatty acid and organic acid esters of monoglycerol, for example, stearic acid and citric acid ester of monoglycerol, stearic acid and acetic acid ester of monoglycerol, stearic acid and succinic acid ester of monoglycerol, caprylic acid and succinic acid ester of monoglycerol, stearic acid and lactic acid ester of monoglycerol, stearic acid and diacetyltartaric acid ester of monoglycerol can be mentioned. As the polyglycerol fatty acid ester, for example, one having an average degree of polymerization of polyglycerin of 2 - 10, wherein the constituent fatty acid has 6 to 22 carbon atoms, can be mentioned, preferably polyglycerin fatty acid monoester. More preferable example is decaglycerol fatty acid monoester, specifically decaglycerol monolaurate, decaglycerol monomyristate, decaglycerol monooleate, decaglycerol monopalmitate, and decaglycerol monostearate. Examples of the polyglycerin condensed ricinoleate include one having an average degree of polymerization of polyglycerin of 2 - 10, wherein the average degree of condensation of polyricinoleic acid (average number of condensation of ricinoleic acid) is 2 to 4.

As the aforementioned sucrose fatty acid esters, one wherein one or more hydroxyl groups of sucrose is/are each esterified with fatty acid having 6 to 22, preferably 12 to 18 , carbon atoms can be mentioned. Further preferred is fatty acid monoester, specifically sucrose monostearate.

Examples of the aforementioned sorbitan fatty acid esters include sorbitans wherein one or more hydroxyl groups thereof are esterified by fatty acids having 6 to 22, preferably 12 to 18, carbon atoms, and polyoxyethylene sorbitan fatty acid esters wherein polyoxyethylene is added to hydroxyl groups of sorbitans, further preferably polyoxyethylenesorbitan monooleate.

Examples of the aforementioned lecithins include egg-yolk lecithin, soybean lecithin, enzymatically decomposed lecithin, phosphatidylcholine, phosphatidylethanolamine, phosphatidylserine, sphingomyelin, dicetyl phosphate, stearylamine, phosphatidylglycerol, phosphatidic acid, phosphatidylinositolamine, cardiolipin, ceramide phosphorylethanolamine, ceramide phosphoryl glycerol and a mixture thereof. Of these, enzymatically decomposed lecithin is preferable. The enzymatically decomposed lecithin is, for example, preferably obtained by reacting phospholipase A₂ with egg-yolk lecithin or soybean lecithin, and one containing lysolecithin as a main ingredient is preferable.

As the aforementioned saponins, for example, enju saponin, quillaja saponin, soybean saponin, yucca saponin and the like can be mentioned.

In the present invention, the content of surfactant (D) in the bioactive substance-containing powder is within the range of 0.1 - 30 wt%, preferably 1 - 25 wt%, more preferably 5 - 20 wt%. When the content of surfactant (D) is less than 0.1 wt%, the emulsifiability is insufficient, and when it exceeds 30 wt%, the powder flowability and recovery rate are degraded.

Moreover, the ratio of water-soluble polymer (B) and a saccharide (C) other than polysaccharide in the powder of the present invention is determined in consideration of the emulsifiability and powder flowability, and is preferably within the range of (B):(C) of 20:1 - 3:1, more preferably 15:1 - 4:1, most preferably 10:1 - 5:1. When the ratio of water-soluble polymer (B) to a saccharide (C) other than polysaccharide is too high, emulsifiability becomes fine but the powder flowability tends to be degraded. Conversely, when the ratio of a saccharide (C) other than polysaccharide is too high, the powder flowability becomes fine but the emulsifiability is degraded.

In addition, the powder of the present invention may comprise various additives and water-soluble active ingredients useful for a wide variety of purposes in foods, cosmetics, and pharmaceuticals, added according to the respective purposes, as long as the properties thereof are not affected.

Examples of such additives include excipients such as crystalline cellulose, calcium phosphate, calcium sulfate, disintegrants such as calcium citrate, calcium carbonate, sodium hydrogencarbonate, dextrin, crystalline cellulose, carboxymethylcellulose, tragacanth, alginic acid, lubricants such as talc, magnesium stearate, polyethylene glycol, silica, and hydrogenated oil, antiblocking agents such as stearic acid, light anhydrous silicic acid, and hydrated silicon dioxide, absorption promoters such as higher alcohols, and higher fatty acids, solubilizing agents such as fumaric acid, succinic acid, and malic acid, stabilizers such as benzoic acid, sodium benzoate, ethyl p-oxybenzoate, and bees wax.

Examples of the water-soluble active ingredients include water-soluble vitamins, amino acids, organic acids, peptides, proteins, nucleic acids, and water-soluble polyphenols.

Examples of the water-soluble vitamins include vitamin C, vitamin B, folic acid, nicotinic acid, nicotinic acid amide, pantothenic acid, pyrroloquinolinequinone, and isomers and derivatives thereof.

Examples of the amino acids include alanine, β-alanine, arginine, asparagine, aspartic acid, cysteine, N-acetylcysteine, selenocysteine, cystine, glutamine, glutamic acid, glycine, histidine, isoleucine, leucine, lysine, pyrrololysine, hydroxylysine, methionine, phenylalanine, proline, hydroxyproline, serine, O-phosphoserine, threonine, tryptophan, tyrosine, valine, thyroxine, desmosine, ornithine, creatine, γ-aminobutyric acid, theanine, taurine, and isomers and derivatives thereof. L-carnitine, a special amino acid, and pharmacologically acceptable salts thereof, such as tartrates and fumarates, acetyl-L-carnitine, and propionyl-L-carnitine are also suitable. It is also suitable to use peptides of two or more of these amino acids bound together; examples include cysteine peptides (ex. glutathione), soybean peptide, sesame peptide, silk peptide, sardine peptide, collagen peptide and casein phosphopeptide. Examples of organic acids other than amino acids include citric acid, malic acid, succinic acid, catechinic acid, picric acid, fumaric acid, maleic acid, and tartaric acid.

The term polyphenol generically refers to a group of plant ingredients having a plurality of phenolic hydroxyl groups (hydroxy groups) in the molecular structure thereof; these ingredients are water-soluble or oil-soluble substances having excellent antioxidant power, contained in most plants as pigments or bitter tastes. Examples of water-soluble polyphenols suitable for use in the present invention include grape polyphenol, pine tree bark polyphenol, apple polyphenol, cacao polyphenol, and green tea polyphenol. These polyphenols occur as multi-ingredient systems; specifically, flavonoids are exemplified by isoflavons such as genistin, daidzein, and puerarin; flavonols such as quercetin, kaempferol, myricetin, and rutin; flavanones such as hesperidin, naringenin, proanthocyanidin, anthocyanin, cyanidin, delphinidin, malvidin, peonidin, and petunidine; flavanol such as epicatechin, epigallocatechin, epicatechin gallate, epigallocatechin gallate, and theaflavine; and flavons such as chrysin, apigenin, and luteolin. Lignans such as sesamin, sesamolin, sesaminol, and sesamol, or chlorogenic acid, gallic acid, ellagic acid, galangin, and fisetin are also suitable.

Furthermore, minerals can be added as other useful ingredients; examples include sodium, calcium, magnesium, zinc, iron, copper, selenium, chromium, manganese, iodine, molybdenum, and salts thereof.

In the present invention, when a bioactive substance to be used is unstable to light, a light-fast ingredient may be further added. A light-fast ingredient should be a component used for foods, quasi-drugs or pharmaceutical products and, for example, colorants used for soft capsules and hard capsules is preferable, with specific preference given to pigment such as titanium oxide, food colors, red iron oxide pigment, safflower pigment, annatto pigment, caramel pigment, gardenia pigment, tar pigment, and chlorophyll. The amount of the light-fast ingredient to be added should not influence the disintegration property and dispersibility/solubility of the obtained powder and is, for example, 0.01 - 10.0 wt%, preferably 0.1 - 5.0 wt%. When the amount of the addition is less than 0.01 wt%, the effect of stability to light may not be achieved, and when it exceeds 10.0 wt%, the disintegration property and dispersibility/solubility of the obtained powder may be affected.

Now, the production method of the powder of the present invention is explained. The bioactive substance-containing powder of the present invention as defined herein is preferably obtained by the following production method.

The powder of the present invention can be obtained by preparing an oil-in-water emulsified composition from an oil component (A) (oil phase) containing coenzyme Q10 as a bioactive substance and optionally at least one further bioactive substance selected from oil-soluble vitamins, plant extract and carotenoids and an aqueous solution containing water-soluble polymer (B), a saccharide (C) as defined herein other than polysaccharide and a surfactant (D) (hereinafter to be indicated as an aqueous phase), and removing water from the oil-in-water emulsified composition.

In the production method of the powder of the present invention, when an oil component (A) containing coenzyme Q10 as a bioactive substance is to be prepared, a method wherein coenzyme Q10 is melted at not less than 50°C, a fat and oil is added as necessary, and the mixture is mixed by stirring and the like is most convenient and preferable. When the coenzyme Q10 to be used is of a reduced type, an operation under a nitrogen atmosphere or under reduced pressure is preferable to prevent oxidation. When a licorice ethanol extract as a further bioactive substance is to be dissolved at a high concentration in fat and oil, heating to about 40°C is preferable to decrease the viscosity. Specific examples and preferable examples of oil component (A) here are the same as those described for the explanation of the above-mentioned bioactive substance-containing powder.

In the production method of the powder of the present invention, an aqueous solution containing water-soluble polymer (B), a saccharide (C) as defined herein other than polysaccharide and surfactant (D) is prepared, and the above-mentioned oil component containing bioactive substance (A) is added to the aqueous solution (aqueous phase) to give an oil-in-water emulsified composition. Specifically, for example, a method wherein water-soluble polymer (B), a saccharide (C) as defined herein other than polysaccharide and surfactant (D) are dissolved in water to give an aqueous solution, oil component (A) containing coenzyme Q10 as a bioactive substance and optionally at least one further bioactive substance selected from oil-soluble vitamins, plant extract and carotenoids, which is heated as necessary, is added to the aqueous phase heated as necessary, and the oil component (A) is dispersed/emulsified ultrafinely to a desired average particle size using a known emulsion device such as a stirring type homomixer, a high-pressure homogenizer etc., is most convenient and preferable. Besides this, the bioactive substance or, where necessary, other oil component may be added to the aqueous phase previously heated as necessary, the bioactive substance or other oil component is melted or dissolved in the aqueous phase and then emulsified.

The particle size of the emulsified bioactive substance-containing oil component (A) as defined herein in the above-mentioned oil-in-water emulsified composition is generally within the range of 10 - 5000 nm, preferably 10 - 1000 nm, more preferably 10 - 800 nm, most preferably 10 - 500 nm. An average particle size of the oil component (A) in the oil-in-water emulsified composition of greater than 5000 nm is unpreferable, since the recovery rate of the emulsified powder decreases in the drying step. On the other hand, an average particle size of the oil component (A) in the oil-in-water emulsified composition of smaller than 10 nm is unpreferable, since an ultra-high pressure in the emulsification step or an emulsifying operation under ultra-high speed stirring for a long time is necessary. The particle size of the emulsified oil component (A) in the oil-in-water emulsified composition can be measured by a commercially available laser diffraction/scattering type particle size distribution measurement device, a dynamic light scattering particle size distribution measurement device and the like.

The emulsification method of the above-mentioned oil-in-water emulsified composition should achieve a desired particle size of the emulsified particles, and a mechanical emulsification method using a general emulsifier can be mentioned. As the apparatus to be used for a mechanical emulsification method, high-speed stirring emulsion machines such as TK homomixer (manufactured by Primix Corporation), Filmics (manufactured by Primix Corporation), Polytron (manufactured by KINEMATICA), Hiscotron (manufactured by microtec nition), Cleamix W-Motion (manufactured by M Technique Corporation) and the like, high-pressure emulsion machines such as microfluidizer (manufactured by Mizuho Industrial Co., Ltd.), Ultimizer system (manufactured by Sugino Machine Limited), nanomizer (manufactured by Yoshida Kikai Co., Ltd.), Manton-Gaulin homogenizer and the like, colloid mill, ultrasonication homogenizer and the like can be mentioned. Besides the mechanical emulsification methods, membrane emulsion method, microchannel emulsion method, natural emulsion method, phase inversion emulsion method, gel emulsion method, and D phase emulsion method can also be utilized. Of these, use of a high-pressure emulsion machine is preferable since it reduces the particle size of the emulsified particles, wherein the homogenized pressure is not less than 10 MPa, preferably not less than 20 MPa, more preferably not less than 50 MPa. The treatment may be performed plural times to afford a desired particle size of the emulsified particles.

In the production method of the powder of the present invention, a step for preparing an oil-in-water emulsified composition from an aqueous phase and oil component (A) containing a bioactive substance, which is coenzyme Q10, is preferably performed at a temperature higher than the melting point of the coenzyme Q10, which is generally within the range of 50 - 100°C, preferably 50 - 90°C, more preferably 60 - 80°C.

It is preferable to handle the above-mentioned oil-in-water emulsified composition at a concentration at which the viscosity of aqueous solution does not exceed 1 Poise, since the transferring property and the like can be ensured.

In the production method of the powder of the present invention, a step for removing water from an oil-in-water emulsified composition is not particularly limited as long as it can remove water. As a general method, a method including drying can be mentioned. For example, spray-drying method, freeze-drying method, vacuum drying method, belt drying method, shelf drying method, drum drying method, liquid-drying method, spray cooler method and the like can be mentioned. Of these, a spray-drying method most widely prevalent is particularly preferable from the aspects of producibility and the like. In the case of a spray-drying method, the spray system is not particularly limited and, for example, 2 fluid nozzle, 3 fluid nozzle and atomizer can be mentioned. In this way, water is removed to give the powder of the present invention. The powder after recovery may be subjected to a classification operation to achieve a particle size desirable as a predetermined product.

The median size of an emulsified particle in the oil-in-water emulsion prepared by dissolving the thus obtained powder of the present invention in water is generally within the range of 10 - 3000 nm, preferably 50 - 2000 nm, more preferably 100 - 2000 nm. In the present invention, the above-mentioned median size can be measured by a method including dissolving the obtained powder(emulsified powder) in water, and measuring the particle size of the obtained aqueous emulsion by a commercially available laser diffraction/scattering type particle size distribution measurement device, a dynamic light scattering particle size distribution measurement device and the like.

An emulsified powder containing coenzyme Q10 as a bioactive substance, which is obtained by the present invention can also be processed into powder preparation, powder, granule, tablet, or pill, and filled in a hard capsule of gelatin, or cellulose, or a soft capsule to give a supplement or a pharmaceutical product. Inasmuch as the powder shows good solubility in water, it can be dissolved in water to give an aqueous solution, which may be used as a drinkable preparation or cosmetic, or directly mixed with general foods, or feed. The powder of the present invention can be used for foods such as general foods, food with nutrient function claims, food for specified health uses, nutritional supplement, nutritional product, and drinks; pharmaceutical products such as therapeutic drug, prophylactic drug, and animal drug; quasi-drug, cosmetic, and feed as mentioned above.

### [Examples]

The present invention is explained in more detail in the following by referring to Examples.

### (Example 1)

Gum arabic (manufactured by Colloïdes Naturels International; Instant Gum AA, 9.6 g), enzymatically decomposed lecithin (manufactured by Cargill; Emultop HL50IP, 1.0 g) and erythritol (manufactured by Mitsubishi-Kagaku Foods, 1.0 g) were dissolved in distilled water (180 g) at 60°C to give an aqueous solution. Separately, oxidized coenzyme Q10 (manufactured by Kaneka Corporation; Kaneka Coenzyme Q10, 8.4 g) was melted at 60°C and added to the above-mentioned aqueous solution at 60°C. The mixture was emulsified by POLYTRON (manufactured by KINEMATICA) at 10000 rpm × 10 min to give an oil-in-water emulsified composition. Then, the composition was sprayed and dried using a spray dryer (B-290; Nihon BUCHI K.K.) under the conditions of a blast temperature of 170°C, an exhaust air temperature of 90°C and a flow rate at 3 ml/min to give an oxidized coenzyme Q10-containing emulsified powder. As the property of the obtained emulsified powder, the flowability was good and attachment of the powder to the inside of the apparatus was extremely small. The obtained emulsified powder was dissolved in water, and the particle size of the obtained aqueous emulsion was measured using PARTICLE SIZE ANALYZER (dynamic light scattering particle size distribution measurement apparatus, LB-550, manufactured by HORIBA). As a result, the median size of the emulsified particles was 824 nm.

### (Example 2)

Using the same materials and method as in Example 1 except that the amount of gum arabic was changed to 7.6 g, the amount of oxidized coenzyme Q10 was changed to 10.4 g, an emulsified powder containing oxidized coenzyme Q10 was obtained. As the property of the obtained emulsified powder, the flowability was good and attachment of the powder to the inside of the apparatus was extremely small. The obtained emulsified powder was dissolved in water, and the particle size of the emulsified particles was measured in the same manner as in Example 1. As a result, the median size was 1046 nm.

### (Example 3)

Using the same materials and method as in Example 1 except that the amount of gum arabic was changed to 11.8 g, the amount of oxidized coenzyme Q10 was changed to 4.2 g, and decaglycerol monopalmitate (manufactured by Mitsubishi-Kagaku Foods; P-8D, 3.0 g) was used instead of the enzymatically decomposed lecithin, an emulsified powder containing oxidized coenzyme Q10 was obtained. As the property of the obtained emulsified powder, the flowability was good and attachment of the powder to the inside of the apparatus was extremely small, and the recovery rate was not less than 60%. The obtained emulsified powder was dissolved in water, and the particle size of the emulsified particles was measured in the same manner as in Example 1. As a result, the median size was 247 nm.

### (Example 4)

Using the same materials and method as in Example 1 except that the amount of gum arabic was changed to 8.6 g, xylitol (manufactured by Towa Kasei, 2.0 g) was used instead of erythritol, an emulsified powder containing oxidized coenzyme Q10 was obtained. As the property of the obtained emulsified powder, the flowability was good and attachment of the powder to the inside of the apparatus was extremely small, and the recovery rate was not less than 60%. The obtained emulsified powder was dissolved in water, and the particle size of the emulsified particles was measured in the same manner as in Example 1. As a result, the median size was 1799 nm.

### (Reference Example 5)

Gum arabic (manufactured by Colloïdes Naturels International; Instant Gum AA, 12.0 g), enzymatically decomposed lecithin (manufactured by Cargill; Emultop HL50IP, 1.0 g) and erythritol (manufactured by Mitsubishi-Kagaku Foods, 1.0 g) were dissolved in distilled water (180 g) at 60°C to give an aqueous solution. Separately, licorice polyphenol-containing oil (manufactured by Kaneka Corporation; KANEKA GLAVONOID, oil component; MCT, licorice polyphenol content; about 30 wt%, 6.0 g) were heated to 60°C, and added to the above-mentioned aqueous solution at 60°C. The mixture was emulsified by POLYTRON (manufactured by KINEMATICA) at 10000 rpm × 10 min to give an oil-in-water emulsified composition. Then, the composition was sprayed and dried using a spray dryer (B-290; Nihon BUCHI K.K.) under the conditions of a blast temperature of 170°C, an exhaust air temperature of 90°C and a flow rate at 3 ml/min to give a licorice polyphenol-containing emulsified powder. As the property of the obtained emulsified powder, the flowability was good and attachment of the powder to the inside of the apparatus was extremely small, and the recovery rate was not less than 60%. The obtained emulsified powder was dissolved in water, and the particle size of the emulsified particles was measured in the same manner as in Example 1. As a result, the median size was 1300 nm.

### (Reference Example 6)

Using the same materials and method as in Example 5 except that the amount of gum arabic was changed to 6.0 g, the amount of licorice polyphenol-containing oil was changed to 12.0 g, an emulsified powder containing licorice polyphenol was obtained. As the property of the obtained emulsified powder, the flowability was good and attachment of the powder to the inside of the apparatus was extremely small, and the recovery rate was not less than 60%. The obtained emulsified powder was dissolved in water, and the particle size of the emulsified particles was measured in the same manner as in Example 1. As a result, the median size was 1300 nm.

### (Reference Example 7)

To each of the bioactive substance-containing emulsified powders (10 g) obtained in Examples 1 - 6 were added cellulose (manufactured by NIHON SHOKUHIN KAKO CO., LTD, 9.8 g) and magnesium stearate (Manufactured by Nacalai Tesque, Inc., 0.2 g), and the mixture was stirred for 1 min. According to general production method of tablet, tablets (300 mg per tablet) were produced. The appearance of each of the obtained tablets was fine and tableting trouble was absent. In addition, problems such as sticking and the like were not found during the production.

### (Comparative Example 1)

Gum arabic (manufactured by Colloïdes Naturels International; Instant Gum AA, 11.6 g) was dissolved in distilled water (180 g) at 60°C to give an aqueous solution. Separately, oxidized coenzyme Q10 (manufactured by Kaneka Corporation; Kaneka Coenzyme Q10, 8.4 g) was melted at 60°C and added to the above-mentioned aqueous solution at 60°C. The mixture was emulsified by POLYTRON (manufactured by KINEMATICA) at 10000 rpm × 10 min to give an oil-in-water emulsified composition. Then, the composition was sprayed and dried using a spray dryer (B-290; Nihon BUCHI K.K.) under the conditions of a blast temperature of 170°C, an exhaust air temperature of 90°C and a flow rate at 3 ml/min to give an oxidized coenzyme Q10-containing emulsified powder. As the property of the obtained emulsified powder, the flowability was comparatively good; however, the recovery rate was less than 60%. The obtained emulsified powder was dissolved in water, and the particle size of the emulsified particles was measured in the same manner as in Example 1. As a result, the median size was 3100 nm and emulsifiability was insufficient.

### (Comparative Example 2)

Gum arabic (manufactured by Colloïdes Naturels International; Instant Gum AA, 8.0 g), decaglycerol monopalmitate (manufactured by Mitsubishi-Kagaku Foods; P-8D, 1.0 g), dextrin (manufactured by Matsutani Chemical Industry Co., Ltd.; Pinedex #2, DE=11±1, 2.6 g) were dissolved in distilled water (180 g) at 60°C to give an aqueous solution. Separately, oxidized coenzyme Q10 (manufactured by Kaneka Corporation; Kaneka Coenzyme Q10, 8.4 g) was melted at 60°C and added to the above-mentioned aqueous solution at 60°C. The mixture was emulsified by POLYTRON (manufactured by KINEMATICA) at 10000 rpm × 10 min to give an oil-in-water emulsified composition. Then, the composition was sprayed and dried using a spray dryer (B-290; Nihon BUCHI K.K.) under the conditions of a blast temperature of 170°C, an exhaust air temperature of 90°C and a flow rate at 3 ml/min to give an oxidized coenzyme Q10-containing emulsified powder. As the property of the obtained emulsified powder, the flowability was poor, attachment of the powder to the inside of the apparatus was high, and the recovery rate was less than 60%. The obtained emulsified powder was dissolved in water, and the particle size of the emulsified particles was measured in the same manner as in Example 1. As a result, the median size was 403 nm.

### (Reference Example 8)

Using the same materials and method as in Example 1 except that vitamin E (manufactured by Riken vitamin Co., Ltd.; E oil 800, 8.4 g) was used instead of oxidized coenzyme Q10, an emulsified powder containing vitamin E was obtained. As the property of the obtained emulsified powder, the flowability was good and attachment of the powder to the inside of the apparatus was extremely small. The obtained emulsified powder was dissolved in water, and the particle size of the emulsified particles was measured in the same manner as in Example 1. As a result, the median size of the emulsified particles was 906 nm.

### (Example 9)

Using the same materials and method as in Example 1 except that reduced coenzyme Q10 (manufactured by Kaneka Corporation; KANEKA QH, 8.4 g) was used instead of oxidized coenzyme Q10, and operating under a nitrogen atmosphere, an emulsified powder containing reduced coenzyme Q10 was obtained. As the property of the obtained emulsified powder, the flowability was good and attachment of the powder to the inside of the apparatus was extremely small. The obtained emulsified powder was dissolved in water, and the particle size of the emulsified particles was measured in the same manner as in Example 1. As a result, the median size of the emulsified particles was 812 nm.

## Claims

1. A bioactive substance-containing emulsified powder comprising 1 - 70 wt% of an oil component (A) comprising coenzyme Q10 as a bioactive substance, 20 - 97.9 wt% of a water-soluble polymer (B), 1 - 20 wt% of a saccharide (C) other than polysaccharides wherein the saccharide (C) is at least one kind of sugar alcohol selected from the group consisting of sorbitol, mannitol, xylitol, erythritol, maltitol, lactitol and palatinit,and 0.1 - 30 wt% of a surfactant (D).

2. A powder according to claim 1, wherein the oil component (A) further comprises at least one further bioactive substance selected from the group consisting of oil-soluble vitamins, plant extract and carotenoids.

3. A powder according to claim 2, wherein the oil-soluble vitamin is at least one kind selected from the group consisting of vitamin A, vitamin D, vitamin E, vitamin K and derivatives thereof.

4. A powder according to claim 2, wherein the plant extract is at least one kind selected from the group consisting of licorice, turmeric, perilla, clove, cinnamon, ginger, lemongrass, peppermint, dokudami (Houttuyania cordata), coix seed, rice bran, cornflower, fennel, boxthorn, zanthoxylum, nasturtium, dioscorea, sanryo, kinkaran (Tinospora capillipes), *amachazuru* (Gynostema pentaphyllum), thuja, hakutouou (Pulsatilla chinensis), parsley, onion, nutmeg, wild-rice, gluten feed, *konnyaku tobiko* (konnyaku byproduct powder), paprika, horseradish, lemon, capsicum, sesame, spearmint and leaf mustard.

5. A powder according to claim 2, wherein the carotenoid is at least one kind selected from the group consisting of carotenes, xanthophylls, and derivatives thereof.

6. A powder according to any one of claims 1 to 5, wherein the water-soluble polymer (B) is at least one kind selected from the group consisting of gum arabic, gum ghatti, gelatine, agar, starch, modified starch, pectin, carrageenan, casein, dried albumen, curdlan, alginic acids, soybean polysaccharide, pullulan, celluloses, xanthan gum, carmellose salt and polyvinylpyrrolidone.

7. A powder according to any one of claims 1 to 6, wherein the surfactant (D) is at least one kind selected from the group consisting of glycerol fatty acid esters, sucrose fatty acid esters, sorbitan fatty acid esters, lecithins and saponins.

8. A powder according to any of claims 1 to 7, wherein the oil component (A) comprises 5 - 100 wt% of a bioactive substance and 0 - 95 wt% of fat and oil.

9. A powder according to claim 8, wherein the fat and oil is medium chain triglyceride.

10. A food, pharmaceutical product, cosmetic or feed comprising a powder according to any one of claims 1 to 9.

## Patentansprüche

1. Bioaktive Substanz enthaltendes emulgiertes Pulver, umfassend 1 bis 70 Gew.-% einer Ölkomponente (A), die Coenzym Q10 als bioaktive Substanz umfasst, 20 bis 97,9 Gew.-% eines wasserlöslichen Polymers (B), 1 bis 20 Gew.-% eines Saccharids (C) ausgenommen Polysaccharide, wobei das Saccharid (C) wenigstens eine Art Zuckeralkohol ist, der aus der Gruppe ausgewählt ist, die aus Sorbit, Mannit, Xylit, Erythrit, Maltit, Lactit und Palatinit besteht, und 0,1 bis 30 Gew.-% eines Tensids (D).

2. Pulver gemäß Anspruch 1, wobei die Ölkomponente (A) weiterhin wenigstens eine weitere bioaktive Substanz umfasst, die aus der Gruppe ausgewählt ist, die aus öllöslichen Vitaminen, Pflanzenextrakt und Carotinoiden besteht.

3. Pulver gemäß Anspruch 2, wobei das öllösliche Vitamin wenigstens eine Art ist, die aus der Gruppe ausgewählt ist, die aus Vitamin A, Vitamin D, Vitamin E, Vitamin K und Derivaten davon besteht.

4. Pulver gemäß Anspruch 2, wobei der Pflanzenextrakt wenigstens eine Art ist, die aus der Gruppe ausgewählt ist, die aus Lakritze, Kurkuma, Perilla, Gewürznelke, Zimt, Ingwer, Zitronengras, Pfefferminz, Eidechsenschwanz (*Houttuyania cordata*), Hiobstränensamen, Reiskleie, Kornblume, Fenchel, Bocksdorn, Zanthoxylum, Brunnenkresse, Yams, Igelkolben, Kinkaran (*Tinospora capillipes*), Jiaogulan (*Gynostema pentaphyllum*), Thuja, *Pulsatilla chinensis,* Petersilie, Zwiebel, Muskat, Wildem Reis, Kleberfutter, Konjakmehl, Paprika, Meerrettich, Zitrone, Chili, Sesam, Grüner Minze und Braunem Senf besteht.

5. Pulver gemäß Anspruch 2, wobei das Carotinoid wenigstens eine Art ist, die aus der Gruppe ausgewählt ist, die aus Carotinen, Xanthophyllen und Derivaten davon besteht.

6. Pulver gemäß einem der Ansprüche 1 bis 5, wobei das wasserlösliche Polymer (B) wenigstens eine Art ist, die aus der Gruppe ausgewählt ist, die aus Gummi arabicum, Ghattigummi, Gelatine, Agar Agar, Stärke, modifizierter Stärke, Pektin, Carrageen, Casein, getrocknetem Albumen, Curdlan, Alginsäuren, Sojapolysaccharid, Pullulan, Cellulosen, Xanthan, Carmellosesalz und Polyvinylpyrrolidon besteht.

7. Pulver gemäß einem der Ansprüche 1 bis 6, wobei das Tensid (D) wenigstens eine Art ist, die aus der Gruppe ausgewählt ist, die aus Glycerinfettsäureestern, Saccharosefettsäureestern, Sorbitanfettsäureestern, Lecithinen und Saponinen besteht.

8. Pulver gemäß einem der Ansprüche 1 bis 7, wobei die Ölkomponente (A) 5 bis 100 Gew.-% einer bioaktiven Substanz und 0 bis 95 Gew.-% Fett und Öl umfasst.

9. Pulver gemäß Anspruch 8, wobei es sich bei dem Fett und Öl um mittelkettige Triglyceride handelt.

10. Lebensmittel, pharmazeutisches Produkt, Kosmetik oder Futter, umfassend ein Pulver gemäß einem der Ansprüche 1 bis 9.

## Revendications

1. Poudre émulsionnée contenant une substance bioactive comprenant 1 à 70 % en poids d'un composant huileux (A) comprenant la coenzyme Q10 en tant que substance bioactive, 20 à 97,9 % en poids d'un polymère hydrosoluble (B), 1 à 20 % en poids d'un saccharide (C) autre que les polysaccharides, lequel saccharide (C) est au moins un type d'alcool de sucre choisi dans le groupe constitué par le sorbitol, le mannitol, le xylitol, l'éryrhtitol, le maltitol, le lactitol et la palatinite, et 0,1 à 30 % en poids d'un tensioactif (D).

2. Poudre selon la revendication 1, dans laquelle le composant huileux (A) comprend en outre au moins une autre substance bioactive choisie dans le groupe constitué par les vitamines liposolubles, les extraits végétaux et les caroténoïdes.

3. Poudre selon la revendication 2, dans laquelle la vitamine liposoluble est au moins un type choisi dans le groupe constitué par la vitamine A, la vitamine D, la vitamine E, la vitamine K et leurs dérivés.

4. Poudre selon la revendication 2, dans laquelle l'extrait végétal est au moins un type choisi dans le groupe constitué par la réglisse, le curcuma, le perilla, la girofle, la cannelle, le gingembre, la citronnelle, la menthe poivrée, le poivre de Chine (Houttuyania cordata), les graines de coïx, le son de riz, le bleuet, le fenouil, le lyciet, le zanthoxylum, la capucine, l'igname, le sanryo, le kinkaran (Tinospora capillipes), le jiaogulan (Gynostema pentaphyllum), le thuya, l'anémone chinoise (Pulsatilla chinensis), le persil, l'oignon, la muscade, le riz sauvage, le gros gluten, le *konnyaku tobiko* (sous-produit en poudre de konjac), le paprika, le raifort, le citron, le poivron, le sésame, la menthe verte et la moutarde en feuille.

5. Poudre selon la revendication 2, dans laquelle le caroténoïde est au moins un type choisi dans le groupe constitué par les carotènes, les xanthophylles, et leurs dérivés.

6. Poudre selon l'une quelconque des revendications 1 à 5, dans laquelle le polymère hydrosoluble (B) est au moins un type choisi dans le groupe constitué par la gomme arabique, la gomme ghatti, la gélatine, la gélose, l'amidon, l'amidon modifié, la pectine, la carraghénane, la caséine, l'albumen séché, le curdlane, les acides alginiques, les polysaccharides de soja, le pullulane, les celluloses, la gomme xanthane, un sel de carmellose, et la polyvinylpyrrolidone.

7. Poudre selon l'une quelconque des revendications 1 à 6, dans laquelle le tensioactif (D) est au moins un type choisi dans le groupe constitué par les esters d'acide gras et de glycérol, les esters d'acide gras et de saccharose, les esters d'acide gras et de sorbitan, les lécithines, et les saponines.

8. Poudre selon l'une quelconque des revendications 1 à 7, dans laquelle le composant huileux (A) comprend 5 à 100 % en poids d'une substance bioactive et 0 à 95 % en poids d'huile et graisse.

9. Poudre selon la revendication 8, dans laquelle l'huile et graisse est un triglycéride à chaîne moyenne.

10. Aliment, produit pharmaceutique, cosmétique ou aliment pour animaux comprenant une poudre selon l'une quelconque des revendications 1 à 9.
